(19) Europäisches Patentamt
European Patent Office
Office européen des brevets

(11) **EP 0 561 225 B1**

(12) **EUROPÄISCHE PATENTSCHRIFT**

(45) Veröffentlichungstag und Bekanntmachung des Hinweises auf die Patenterteilung:
**19.06.1996 Patentblatt 1996/25**

(51) Int. Cl.$^6$: **C07C 263/10**, C07C 263/20, C08G 18/70

(21) Anmeldenummer: 93103415.1

(22) Anmeldetag: 03.03.1993

(54) **Verfahren zur Herstellung von Isocyanaten oder Isocyanatgemischen und deren Verwendung zur Herstellung von Polyurethanschaumstoffen**

Process for the preparation of isocyanates or isocyanate mixtures and their use in the preparation of polyurethane foams

Procédé pour la préparation d'isocyanates ou mélanges d'isocyanates et leur utilisation dans la préparation de mousses de polyuréthane

(84) Benannte Vertragsstaaten:
**BE DE ES FR GB IT NL PT**

(30) Priorität: **16.03.1992 DE 4208359**

(43) Veröffentlichungstag der Anmeldung:
**22.09.1993 Patentblatt 1993/38**

(73) Patentinhaber: **BAYER AG**
**51368 Leverkusen (DE)**

(72) Erfinder:
• **Gallus, Manfred, Dr.**
  **W-4150 Krefeld (DE)**
• **Gebauer, Herbert, Dr.**
  **W-4150 Krefeld (DE)**
• **Immel, Otto, Dr.**
  **W-4150 Krefeld (DE)**

(56) Entgegenhaltungen:
EP-A- 0 114 970      EP-A- 0 446 781
DE-A- 1 568 623      GB-A- 2 207 671
US-A- 4 876 380

**Beschreibung**

Die Erfindung betrifft ein Verfahren zur Herstellung von Isocyanaten oder Isocyanatgemischen, die keine nennenswerten farbgebenden Komponenten aufweisen, sowie deren Verwendung zur Herstellung von hellen Polyurethanhartschaumstoffen.

Die Beeinflussung der Farbe eines Polyurethanhartschaumes ist Grund vieler Versuche und Arbeiten. Erwünscht sind möglichst hellgelbe bis weiße Farbtöne. Neben dem besseren visuellen Eindruck lassen helle Schäume auf Rohstoffe mit hohem Reinheitsgrad schließen. Bei dunkelgelbem oder graustichigem Ausgangsmaterial bilden sich an der Hartschaumoberfläche unerwünschte Schlieren, die beim Aufsteigen während des Schaumvorganges gebildet werden. Die genaue Zusammensetzung der die Polyurethanschaumfarben verursachenden Farbstoffe oder farbgebenden Komponenten im Polymer ist im einzelnen noch nicht nachgewiesen worden. Wie die Literatur zeigt, ist die Beseitigung der Farbprobleme Bestandteil der Isocyanatherstellung. Eine Vielzahl von Verfahren zur Verbesserung der Polyurethanschaumfarben wird beschrieben. So wird im Patent GB-A-2.207.671 die Verwendung von getrockneten Magnesiumsilikaten zur Entfernung der Spuren an "farbbildenden Verunreinigungen" im Diisocyanatodiphenylmethan (MDI) bei ca. 190°C beansprucht. In US 4.465.639 wird das gebildete "dark colored material", welches bei der Abtrennung des Lösungsmittels bei der Diisocyanatodiphenylmethan-Herstellung entsteht, durch Zugabe von Wasser in geringen Mengen reduziert. In US 4.876.380 wird ein Extraktionsverfahren beschrieben, bei dem Polyisocyanate mittels Methylendichlorid und Pentan in eine "purified fraction by greatly improved color" und eine Fraktion "where color is not a critical requirement" getrennt werden. Die Reinigung von Polyisocyanaten durch Extraktion beschreibt auch EP 0.133.538.

In EP 0.446.781 A1 wird ein Verfahren zur Verbesserung des Polyurethanschaums beschrieben, welches dadurch gekennzeichnet ist, daß Diaminodiphenylmethan (MDA) einer katalytischen Hydrierung in Gegenwart von Hydrierkatalysatoren und einem bestimmten Gehalt an Wasser durchgeführt wird, wobei als Hydrierkatalysatoren sämtliche für die katalytische Hydrierung bekannten Katalysatoren in Betracht kommen. Solch ein Verfahren birgt jedoch den Nachteil in sich, daß bei dieser Hydrierung Nebenprodukte entstehen, die im MDA verbleiben und bei ungünstigen Phosgenierbedingungen trotzdem zu farbgebenden Verunreinigungen im MDI führen. Außerdem ist allgemein bekannt, daß allein durch die Phosgenierungsreaktion die Entstehung von stark gefärbten Verbindungen im ppm-Bereich nicht auszuschließen ist.

Aufgabe war es daher, ein Verfahren zur Verfügung zu stellen, daß es gestattet, Isocyanate oder Isocyanatgemische herzustellen, die keine nennenswerten farbgebenden Komponenten aufweisen, so daß damit helle Polyurethanschaumstoffe hergestellt werden können und wobei die übrigen chemischen und physikalischen Eigenschaften des Isocyanates oder Isocyanatgemisches, wie z.B. NCO-Gehalt und Viskosität, nicht verändert werden.

Diese Aufgabe konnte mit dem erfindungsgemäßen Verfahren gelöst werden.

Gegenstand der vorliegenden Erfindung ist ein Verfahren zur Herstellung von Isocyanaten oder Isocyanatgemischen, die keine nennenswerten farbgebenden Komponenten aufweisen, welches dadurch gekennzeichnet ist, daß die Isocyanate oder Isocyanatgemische nach der Phosgenierung der entsprechenden Amine einer Wasserstoffbehandlung bei einem Druck von 3 bis 150 bar und einer Temperatur von 100 bis 180°C unter Verwendung von Katalysatoren 15 Minuten bis 4 Stunden unterworfen werden.

Bevorzugt wird die Wasserstoffbehandlung bei einem Druck von 30 bis 100 bar durchgeführt.

Als Temperatur wird vorzugsweise 140 bis 160°C bei der Wasserstoffbehandlung eingestellt.

Besonders bevorzugt werden als Katalysatoren Platin, Palladium, Ruthenium, Rhodium, Chrom, Mangan, Kobalt, Nickel, Silber, Gold oder Gemische davon in einer Menge von 0,1 bis 6,0 Gew.-%, bezogen auf das Gesamtgewicht an Katalysatorträger, eingesetzt.

Die verwendeten Katalysatoren werden vorzugsweise auf Trägern wie Aktivkohle, Aluminiumoxid, Spinellen oder Mischoxiden mit einer spezifischen Oberfläche von 8 bis 1.000 $m^2/g$ eingesetzt.

Derartige Spinelle oder Mischoxide können z.B. $Me^{II}Me^{III}_2O_4$ und $Me^{I}_2Me^{III}_2O_4$ mit $Me^I$=Li, $Me^{II}$=Mn, Zn, Co, Fe und Mg und $Me^{III}$= Al, Cr und Fe sein.

Die Katalysatoren werden vorzugsweise als wäßrige Salzlösungen der Metalle auf den Träger aufgetragen, im Vakuum getrocknet und bei Temperaturen von >300°C im Wasserstoffstrom zum Metall reduziert.

Anstelle von Wasserstoff können auch Wasserstoff/Inertgasmischungen mit einem Volumenanteil an Wasserstoff von 3 bis 90 Vol.-% eingesetzt werden.

Es werden vorzugsweise lösungsmittelfreie Isocyanate oder Isocyanatgemische mit einer Viskosität von 8 bis 4.000 mPa.s bei 25°C oder Lösungen von Isocyanaten oder Isocyanatgemischen in Monochlorbenzol eingesetzt.

Bevorzugt werden Isocyanate oder Isocyanatgemische der Diphenylmethanreihe, Toluylendiisocyanate oder Gemische davon der Wasserstoffbehandlung unterzogen.

Da katalytische Hydrierungen (EP 372 993) von organischen Verbindungen vielfach bei hohen Temperaturen (>200°C), hohem Wasserstoffdruck (200 bis 390 bar) und hohem Wasserstoffverbrauch stattfinden, handelt es sich bei dem vorliegenden Verfahren, bei dem ein kaum meßbarer Wasserstoffverbrauch vorliegt, um keine allgemein bekannte Hydrierung.

Vorzugsweise kann zur Entfernung der farbgebenden Verunreinigungen auch mit einem Gemisch von z.B. 5 % Wasserstoff und Rest Inertgas wie z.B. Stickstoff bei Drucken von 3 bis 5 bar gearbeitet werden. Dadurch ist eine Betreibung der Anlage außerhalb des Explosionsbereichs des Wasserstoffs möglich. Außerdem müssen nicht Materialien zur Herstellung der Anlage verwendet werden, die gegen hohen Wasserstoffdruck beständig sind.

Die nach dem erfindungsgemäßen Verfahren hergestellten Isocyanate oder Isocyanatgemische werden zur Herstellung von hellen Polyurethanschaumstoffen verwendet.

Die Erfindung soll anhand der nachfolgenden Beispiele näher erläutert werden.

Versuchsdurchführung

A) Die Wasserstoffbehandlung erfolgt in einem 250 ml-Schüttelautoklaven mit 25 ml Katalysator und 100 ml MDI. Der Wasserstoffdruck im Autoklaven ist von 200 bar bis 6 bar variierbar. Die Produkttemperatur kann von 20 bis 200°C eingestellt werden. Nach Befüllen des Autoklaven mit MDI wird der Wasserstofffülldruck eingestellt, der Autoklav verschlossen und während ca. 45 bis 60 Minuten aufgeheizt, dann ca. 45 bis 60 Minuten auf der Temperatur gehalten. Danach wird während ca. 45 bis 60 Minuten abgekühlt. Während der dreistündigen Behandlung tritt eine Volumenausdehnung auf, die zu einem höheren $H_2$-Druck im Autoklaven führt.

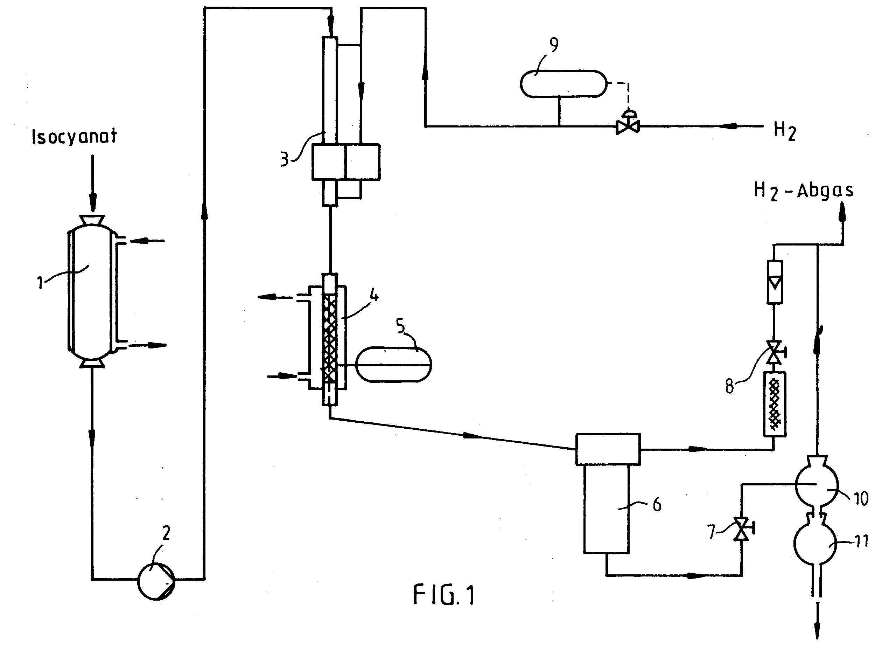

B) Eine kontinuierlich betriebene Wasserstoffbehandlung kann z.B. in der in Figur 1 dargestellten Apparatur durchgeführt werden. Isocyanat wird aus dem Vorratsbehälter 1 über die Druckpumpe 2 in Dosiervorrichtung 3 gepumpt. Der Wasserstoff wird über das Drucksteuerventil 9 von einem Vorratsbehälter auf den gewünschten Druck reduziert. Beide Ströme werden durch den Reaktionsbehälter 4, welcher mit 60 ml Katalysator gefüllt ist und bis 200°C über eine Regeleinrichtung 5 geheizt werden kann, geleitet. Das mit Wasserstoff behandelte Isocyanat wird im Abscheider 6 abgetrennt und über Behälter 10 und 11 ausgeschleust. Der Wasserstoff wird über ein Ventil 8 entspannt und kann als Abgas entfernt werden oder im Kreis gefahren werden.

Analytische Farbbestimmungen des MDI

Die Beurteilung der Farbwerte des polymeren MDI und deren Änderungen nach der Wasserstoffbehandlung erfolgt durch die Messung der Extinktionswerte bei E = 520 nm und E = 430 nm, wobei eine Lösung von 2 g MDI in 100 ml Monochlorbenzol in einem Fotometer z.B. Digital Photometer LP 1W der Fa. Lange GmbH, Berlin vermessen werden. Diese Methode eignet sich zur schnellen Beurteilung von MDI. Die Farbbeurteilung von monomeren MDI erfolgt durch Bestimmung der Hazenfarbzahl (DIN 53 409).

Eine genauere Bestimmungsmethode ergibt sich durch die Messung der Lichtdurchlässigkeit der jeweiligen MDI-Probe in einer Schichtdicke von 1 mm gegen Licht. Anstelle der Extinktion wird das Lichtdurchlässigkeitsspektrum vermessen. Hierbei lassen sich bei den unterschiedlichen Wellenlängen die prozentuale Lichtdurchlässigkeit einer unbehandelten und mit Wasserstoff behandelten Probe besser vergleichen. Bei dieser Bestimmungsmethode arbeitet man lösungsmittelfrei. Man kann bei einem Vergleich der Spektren den Abbau der farbgebenden Komponenten durch die in Prozent ausgedrückte höhere Lichtdurchlässigkeit angeben. Die Lichtdurchlässigkeit kann als Maßstab für den Gehalt an farbgebenden Verunreinigungen herangezogen werden, da bekanntlich farblose Flüssigkeiten praktisch eine 100 %ige Lichtdurchlässigkeit aufweisen.

Isocyanate

Beispiel 1

Ein im Handel erhältliches MDI mit einer Viskosität von 200 ± 20 mPa.s bei 25°C und einem NCO-Gehalt von 31,5 %.

Beispiel 2

Es wurde ein Isocyanat ausgewählt, das eine überdurchschnittliche Farbvertiefung im entsprechenden Polyurethanhartschaum aufweist. Es handelt sich um ein Anfahrprodukt. Das MDI weist einen Anteil von 90 % Zweikern-MDI und davon einen Anteil von ca. 50 % 4,4'-MDI, ca. 40 % 2,4'-MDI und ca. 10 % 2,2'-MDI auf. Die Viskosität beträgt ca. 25 mPa.s bei 25°C und der NCO-Gehalt 32,0 %.

Beispiel 3

Ein nach bekannten Verfahren hergestelltes MDA mit ca. 50 bis 55 % Zweikern-MDA wird in o-Dichlorbenzol als Lösungsmittel phosgeniert. Die Aufarbeitung der phosgenierten Rohprodukte bzw. Abtrennung des Lösungsmittels vom MDI erfolgt in einer Trennkolonne, wobei das gesamte Lösungsmittel o-Dichlorbenzol über Kopf abdestilliert wird. Viskosität des MDI: 320 mPa.s bei 25°C, NCO-Gehalt: 30,7 %.

Beispiel 4

Ein bei der Toluylendiisocyanatherstellung anfallender Destillationsrückstand wird durch Vermischen mit MDI aufbereitet. Das entstehende lösliche TDI-MDI-Gemisch weist eine Viskosität von 520 mPa.s bei 25°C und einen NCO-Gehalt von ca. 28,5 % auf. Ohne Behandlung ergibt das Gemisch eine dunkelbraun-graue Polyurethan-Hartschaumfarbe.

Beispiel 5

Aus einem MDI mit ca. 70 % Zweikern-MDI werden ca. 30 % monomeres MDI mit einem Gehalt an 4,4'-MDI von ca. 94 % und einem Gehalt an 2,4'-MDI von ca. 5,5 % und einem Gehalt an 3-Kern-MDI von ca. 0,5 % im Vakuum abgetrennt. Dieses rohe monomere MDI wird in Monochlorbenzol im Verhältnis 1 : 1 gelöst. Die 50 %ige MDI-Chlorbenzol-Lösung wird einer Wasserstoffbehandlung unterworfen.

Katalysatoren

Beispiel 1

200 g eines handelsüblichen $\alpha$-$Al_2O_3$ mit einer spezifischen Oberfläche von 8 m²/g und einem Kugeldurchmesser von 3 bis 6 mm wurden mit einer Lösung getränkt, die aus
12,4 g $Co(NO_3)_2$ x $6H_2O$
18,3 g $Mn(NO_3)_2$ x $4H_2O$
3,2 g Ag $NO_3$ und
86 g Wasser
hergestellt worden war.
Das getränkte Aluminiumoxid wurde 18 Stunden unter Wasserstrahlvakuum bei 120°C getrocknet und anschließend 3 Stunden bei 400°C getempert. Der so erhaltene Katalysatorträger wurde mit einer Lösung getränkt, die aus 6,52 g $Ru(NO_3)_3$ und 75 g Wasser hergestellt worden war.
Nach einer erneuten Trocknung wurde der Katalysator 3 Stunden im Wasserstrom bei 300°C reduziert. Anschließend wurde der Katalysator mit destilliertem Wasser nitratfrei gewaschen und dann nochmals getrocknet.

Beispiel 2

Als Katalysator wurde eine mit 5 Gew.-% Pd imprägnierte Aktivkohle gewählt. Zur Herstellung des Pd-Katalysators wurde ein Aktivkohle-Granulat von 4 mm eingesetzt, das auf Steinkohlenbasis hergestellt worden war und eine BET-Oberfläche von 1.000 m²/g aufwies.

Beispiel 3

150 g eines handelsüblichen $\alpha$-$Al_2O_3$ mit einer spezifischen Oberfläche von 8 m²/g und einem Kugeldurchmesser von 3 bis 6 mm wurden mit einer Lösung getränkt, die aus
3,7 g $H_2PtCl_6$-Lösung (30 % Pt)
3,1 g Ru $Cl_3$ und
62 g Wasser
hergestellt worden war. Das getränkte Aluminiumoxid wurde 18 Stunden bei 120°C getrocknet und anschließend 3 Stunden bei 370°C im Wasserstoffstrom reduziert. Danach war der Katalysator einsatzbereit.

Beispiel 4

200 g eines handelsüblichen $\gamma$-$Al_2O_3$ mit einer spezifischen Oberfläche von 350 m²/g und einem Kugeldurchmesser von 2 bis 5 mm wurden mit einer Lösung getränkt, die aus
6,68 g H $AuCl_4$-Lösung (30 %) und
70 g Wasser
hergestellt worden war.
Das getränkte Aluminiumoxid wurde im Wasserstrahlvakuum bei 120°C getrocknet und anschließend 3 Stunden bei 380°C im Wasserstoffstrom aktiviert.

Anschließend wurde der Katalysator mit einer Lösung getränkt, die aus 2 g NaOH und 35 g Wasser hergestellt worden war. Nach einer weiteren Trocknung war der Katalysator einsatzbereit.

Versuche

Beispiel 1

123 g des Isocyanates aus Beispiel 1 werden in dem oben beschriebenen Autoklaven zusammen mit 25 ml Katalysator aus Beispiel 1 gefüllt und 3 Stunden mit Wasserstoff bei einem Druck von 30 bar und 160°C behandelt.

Beispiel 2

Das Isocyanat aus Beispiel 2 wird in der aus Figur 1 ersichtlichen Anlage in einem kontinuierlich verlaufenden Versuch mit Wasserstoff behandelt. Die Menge des Katalysators aus Beispiel 2 betrug 60 ml (23,5 g). Während des kontinuierlichen Versuches wurde bei durchschnittlich 120 bar Wasserstoffdruck und einer Wasserstoffdurchlaufmenge von 30 l/h gearbeitet. Die Temperatur des MDI betrug 140°C. Die durchgesetzte Menge belief sich auf ca. 35 g MDI/h.

Beispiel 3

123 g des Isocyanates aus Beispiel 3 werden in dem oben beschriebenen Autoklaven bei 100 bar Wasserstoffdruck und 160°C in Gegenwart von 25 ml des Katalysators aus Beispiel 3 behandelt.

Beispiel 4

123 g des Isocyanates aus Beispiel 4 werden mit 25 ml des Katalysators aus Beispiel 1 bei 170°C und einem Wasserstoffdruck von 60 bar in dem oben beschriebenen Autoklaven behandelt.

Beispiel 5

100 ml einer 50 %igen Lösung von monomerem MDI in Chlorbenzol werden bei 140°C und 30 bar Wasserstoff in dem oben beschriebenen Autoklaven mit Katalysator aus Beispiel 4 behandelt.

Die anfallende Chlorbenzollösung wird im Wasserstrahlvakuum unter Stickstoff chlorbenzolfrei destilliert und bei 1 mbar und ca. 180°C destilliert.

Die gleiche 50 %ige Lösung von Monomer-MDI in Chlorbenzol wurde direkt auf gleiche Weise wie oben chlorbenzolfrei destilliert und anschließend bei 1 mbar und ca. 180°C destilliert. Die Farbbeurteilung erfolgt durch Bestimmung der Hazenfarbzahl, wobei eine 50 %ige Lösung des Produktes in Monochlorbenzol zugrunde liegt.

| monomeres MDI, destilliert | = 80 Hazen |
|---|---|
| monomeres MDI, wasserstoffbehandelt, destilliert | = 40 Hazen |

**Tabelle 1**

| Isocyanat aus | Extinktion ohne Wasserstoff-Behandlung | | Extinktion mit Wasserstoff-Behandlung | | Lichtdurchlässigkeit ohne Wasserstoff-Behandlung in [%] | | | | Lichtdurchlässigkeit mit Wasserstoff-Behandlung in [%] | | | | Hazenfarbzahl ohne Wasserstoff-Behandlung | Hazenfarbzahl mit Wasserstoff-Behandlung |
|---|---|---|---|---|---|---|---|---|---|---|---|---|---|---|
| | E 430 | E 520 | E 430 | E 520 | 450nm | 500nm | 550nm | 600nm | 450nm | 500nm | 550nm | 600nm | | |
| Beispiel 1 | 0,28 | 0,046 | 0,21 | 0,020 | 10 | 42 | 63 | 74 | 13 | 50 | 72 | 78 | | |
| " 2 | 0,575 | 0,440 | 0,068 | 0,013 | 3,5 | 6 | 17,3 | 40 | 30 | 63 | 74 | 80 | | |
| " 3 | 0,869 | 0,253 | 0,487 | 0,088 | 0 | 1,7 | 6,6 | 20 | 0,6 | 14,5 | 40 | 60 | | |
| " 4 | 1,352 | 0,435 | 1,068 | 0,277 | | | | | | | | | | |
| " 5 | | | | | | | | | | | | | 80 | 40 |

EP 0 561 225 B1

**Patentansprüche**

1.  Verfahren zur Herstellung von Isocyanaten oder Isocyanatgemischen, die keine farbgebenden Komponenten aufweisen, dadurch gekennzeichnet, daß die Isocyanate oder Isocyanatgemische nach der Phosgenierung der entsprechenden Amine einer Wasserstoffbehandlung bei einem Druck von 3 bis 150 bar und einer Temperatur von 100 bis 180°C unter Verwendung von Katalysatoren 15 Minuten bis 4 Stunden unterworfen werden.

2.  Verfahren zur Herstellung von Isocyanaten oder Isocyanatgemischen gemäß Anspruch 1, dadurch gekennzeichnet, daß die Isocyanate oder Isocyanatgemische bei einem Druck von 30 bis 100 bar mit Wasserstoff behandelt werden.

3.  Verfahren gemäß Anspruch 1 oder 2, dadurch gekennzeichnet, daß die Wasserstoffbehandlung bei einer Temperatur von 140 bis 160°C stattfindet.

4.  Verfahren gemäß einem der Ansprüche 1 bis 3, dadurch gekennzeichnet, daß als Katalysator Platin, Palladium, Ruthenium, Rhodium, Chrom, Mangan, Kobalt, Nickel, Silber, Gold oder Gemische davon in einer Menge von 0,1 bis 6,0 Gew.-%, bezogen auf das Gesamtgewicht des Katalysatorträgers, eingesetzt werden.

5.  Verfahren gemäß einem der Ansprüche 1 bis 4, dadurch gekennzeichnet, daß als Katalysatorträger Aktivkohle, Aluminiumoxid, Spinelle oder Mischoxide mit einer spezifischen Oberfläche von 8 bis 1000 $m^2/g$ eingesetzt werden.

6.  Verfahren gemäß einem der Ansprüche 1 bis 5, dadurch gekennzeichnet, daß anstelle von Wasserstoff, Wasserstoff/Inertgasmischungen mit einem Volumenanteil an Wasserstoff von 3 bis 90 Vol.-% eingesetzt werden.

7.  Verfahren gemäß einem der Ansprüche 1 bis 6, dadurch gekennzeichnet, daß lösungsmittelfreie Isocyanate oder Isocyanatgemische mit einer Viskosität von 8 bis 4.000 mPa.s bei 25°C oder Lösungen von Isocyanaten oder Isocyanatgemischen in Monochlorbenzol eingesetzt werden.

8.  Verfahren gemäß einem der Ansprüche 1 bis 7, dadurch gekennzeichnet, daß Isocyanate oder Isocyanatgemische der Diphenylmethanreihe, Toluylendiisocyanate oder Gemische davon eingesetzt werden.

**Claims**

1.  A process for the preparation of isocyanates or isocyanate mixtures which possess no colour-imparting components, characterised in that the isocyanates or isocyanate mixtures, after phosgenation of the corresponding amines, are subjected to a hydrogen treatment for 15 minutes to 4 hours at a pressure of 3 to 150 bar and a temperature of 100 to 180°C using a catalyst.

2.  A process for preparing isocyanates or isocyanate mixtures according to Claim 1, characterised in that the isocyanates or isocyanate mixtures are treated with hydrogen at a pressure of 30 to 100 bar.

3.  A process according to Claim 1 or 2, characterised in that hydrogen treatment takes place at a temperature of 140 to 160°C.

4.  A process according to one of Claims 1 to 3, characterised in that platinum, palladium, ruthenium, rhodium, chromium, manganese, cobalt, nickel, silver, gold or mixtures thereof are used as catalyst in an amount of 0.1 to 6.0 wt.%, with respect to the total weight of catalyst support.

5.  A process according to one of Claims 1 to 4, characterised in that activated carbon, aluminium oxide, spinels or mixed oxides with a specific surface area of 8 to 1000 $m^2/g$ are used as catalyst supports.

6.  A process according to one of Claims 1 to 5, characterised in that instead of hydrogen, hydrogen/inert gas mixtures with a proportion by volume of hydrogen of 3 to 90 vol.-% are used.

7.  A process according to one of Claims 1 to 6, characterised in that solvent-free isocyanates or isocyanate mixtures with a viscosity of 8 to 4,000 mPa.s at 25°C or solutions of isocyanates or isocyanate mixtures in monochlorobenzene are used.

8.  A process according to one of Claims 1 to 7, characterised in that isocyanates or isocyanate mixtures from the diphenylmethane series, toluylene diisocyanates or mixtures thereof are used.

**Revendications**

1. Procédé de préparation d'isocyanates ou de mélanges d'isocyanates exempts de composants colorants, caractérisé en ce que, après la phosgénation des amines appropriées, les isocyanates ou mélanges d'isocyanates sont traités à l'aide d'hydrogène en présence de catalyseurs pendant 15 minutes à 4 heures, sous une pression de 3 à 150 bar et à une température de 100 à 180 °C.

2. Procédé de préparation d'isocyanates ou de mélanges d'isocyanates selon la revendication 1, caractérisé en ce que les isocyanates ou mélanges d'isocyanates sont traités à l'hydrogène sous une pression de 30 à 100 bar.

3. Procédé selon la revendication 1 ou 2, caractérisé en ce que le traitement à l'hydrogène est réalisé à une température de 140 à 160 °C.

4. Procédé selon l'une quelconque des revendications 1 à 3, caractérisé en ce que le catalyseur mis en oeuvre est du platine, du palladium, du ruthénium, du rhodium, du chrome, du manganèse, du cobalt, du nickel, de l'argent, de l'or ou des mélanges de ces éléments, dans une quantité de 0,1 à 0,6 % en poids, rapporté au poids total du support de catalyseur.

5. Procédé selon l'une quelconque des revendications 1 à 4, caractérisé en ce que le support de catalyseur mis en oeuvre est du charbon actif, de l'oxyde d'aluminium, des spinelles ou des oxydes mixtes présentant une surface spécifique de 8 à 1000 $m^2$/g.

6. Procédé selon l'une quelconque des revendications 1 à 5, caractérisé en ce que, à la place d'hydrogène, des mélanges hydrogène/gaz inerte comprenant une proportion d'hydrogène de 3 à 90 % en volume sont mis en oeuvre.

7. Procédé selon l'une quelconque des revendications 1 à 6, caractérisé en ce que des isocyanates ou des mélanges d'isocyanates exempts de solvant et présentant une viscosité de 8 à 4000 mPa.s à 25 °C ou des solutions d'isocyanates ou de mélanges d'isocyanates dans le monochlorobenzène sont mis en oeuvre.

8. Procédé selon l'une quelconque des revendications 1 à 7, caractérisé en ce que les isocyanates ou mélanges d'isocyanates mis en oeuvre sont des substances de la série des diphénylméthanes, des toluylènediisocyanates ou leurs mélanges.

FIG. 1

Isocyanat

1

2

3

4

5

6

7

8

9

10

11

H₂

H₂-Abgas